# EUROPEAN PATENT SPECIFICATION

(11) **EP 0 412 760 B1**
(45) Date of publication and mention of the grant of the patent: **23.11.1994**
(21) Application number: 90308646.0
(22) Date of filing: 06.08.1990
(51) Int. Cl.: C07D 405/04, A61K 31/445

(54) **Benzopyran derivatives**
Benzopyran-Derivate
Dérivés de benzopyrannes

(30) Priority: 10.08.1989 GB 8918298
(43) Date of publication of application: 13.02.1991
(73) Proprietor: Beecham Group p.l.c., Brentford, Middlesex TW8 9BD (GB)
(72) Inventor: Evans, John Morris, SmithKline Beecham, The Pinnacles, Harlow, Essex CM19 5AD (GB); Stemp, Geoffrey, SmithKline Beecham, The Pinnacles, Harlow, Essex CM19 5AD (GB); Cassidy, Frederick, SmithKline Beecham, The Pinnacles, Harlow, Essex CM19 5AD (GB); Burrell, Gordon, Cumbria (GB)
(74) Representative: Tocher, Pauline

(56) References cited:
- EP-A- 0 250 077

## Description

This invention relates to a novel benzopyran having pharmacological activity, to a process for its preparation and to its use as a pharmaceutical.

EP-A-250077 (Beecham Group p.l.c.) describes a group of 4-amidobenzopyrans in which the 6-position is substituted by inter alia a C₁₋₆ alkyl group. Example 1 describes the compound wherein the 4-substitutent is 2-oxopiperidinyl and the 6-substituent is methyl, (E1): 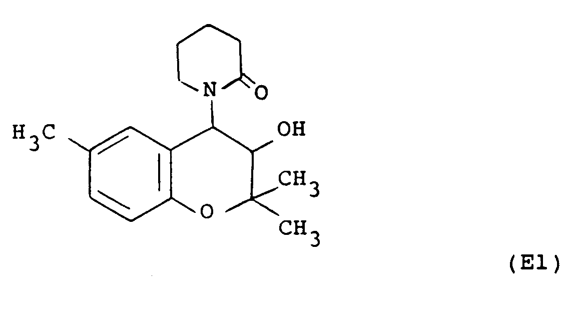
Example 13 describes the compound wherein the 4-substituent is 2-oxopyrrolidinyl and the 6-substituent is ethyl, (E13): 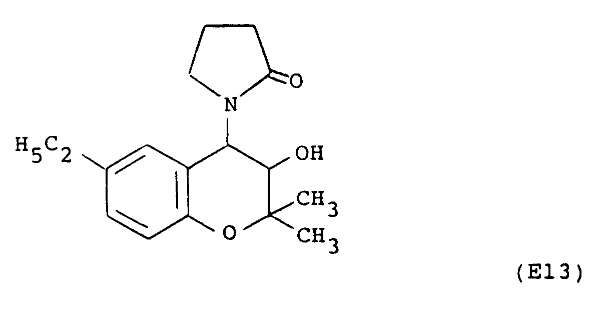
In both E1 and E13 the lactam and OH moieties are trans and the compounds are isolated as racemic mixtures of the (3S,4R)- and (3R,4S)-isomers.

A novel compound not specifically described in EP-A-250077 has now been discovered, this compound having blood pressure lowering activity, useful in the treatment of hypertension, and also bronchodilator activity, useful in the treatment of respiratory tract disorders. In addition, this compound is believed to be a K⁺ channel activator which indicates that is is of potential use in the treatment of disorders associated with smooth muscle contraction of the gastro-intestinal tract, respiratory system, uterus or urinary tract including the ureter. Such disorders include irritable bowel syndrome and diverticular disease; reversible airways obstruction including asthma; premature labour; and incontinence, renal cholic and disorders associated with kidney stones. It is also indicated as of potential use in the treatment of cardiovascular disorders other than hypertension, such as congestive heart failure, angina, peripheral vascular disease, cerebral vascular disease, pulmonary hypertension and right heart failure. It may also be of potential use in the treatment of epilepsy.

The present invention provides the (3S,4R)-isomer of a compound of formula (I): 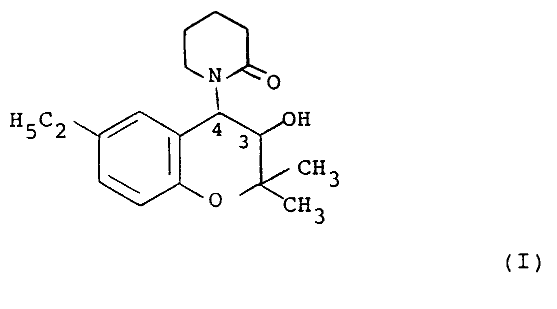
wherein the lactam and OH moieties are trans.

The compound of formula (I) may exist as solvates such as hydrates and these are included wherever a compound of formula (I) is herein referred to.

The compound of formula (I) may be prepared as generally described in EP-A-250077.

The invention further provides a process for the preparation of a compound of formula (I), which process comprises the reaction of a compound of formula (II): 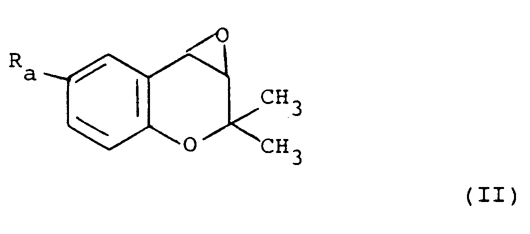
wherein Rₐ is ethyl or a group or atom convertible thereto; with either
i) a compound of formula (III): 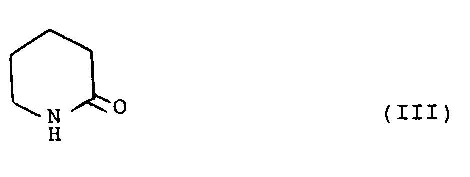 in the form of the piperidinone anion; or
ii) with ammonia to give a trans aminoalcohol of formula (IV): 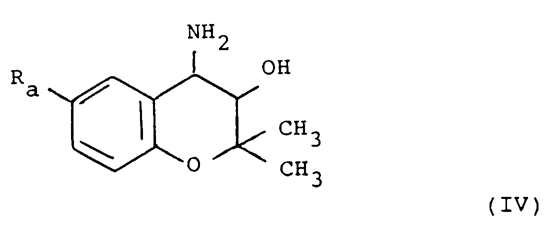 which is subsequently acylated with a compound of formula (V):

   L₁(CH₂)₄COL₂ (V)

   wherein L₁ and L₂ are leaving groups; and thereafter cyclising the resulting compound where necessary;
   and thereafter converting Rₐ (when other than ethyl) to ethyl.

The compound of formula (III) is preferably generated in the form of the piperidinone anion using a base, such as sodium hydride or potassium t-butoxide.

Suitable values for L₁ and L₂ include halo, such as chloro, and (for L₂), hydroxy, C₁₋₄ alkoxy or C₁₋₄ alkanoyloxy (i.e. the compound of formula (V) is a mixed anhydride).

Suitable reagents and conditions for reactions i) and ii) are described in EP-A-250077.

Rₐ when cyano may be converted to ethyl by reduction of the cyano to a formyl group using for example, Raney Nickel, then a Wittig reaction using Ph₃P⁺MeBr⁻ to give the 6-ethenyl derivative which is converted to ethyl by reduction, preferably using palladium on charcoal.

The compound of formula (I) may either be prepared as a racemic mixture of (3S,4R)- and (3R,4S)-isomers which is subsequently resolved according to the methods disclosed in EP-A-120428, or stereospecifically using resolved intermediates. The compound of formula (I) may be prepared from a resolved aminoalcohol of formula (IV) where Rₐ is cyano which is subsequently converted to ethyl as described above. Preferably, Rₐ is ethyl in formula (IV), as described in the Example (Method 2) hereinafter.

Compounds of formulae (II) and (IV) are known or prepared by analogous methods to those used for structurally similar known compounds. They may be prepared in the manner described in EP-A-76075 and 250077, in the name of Beecham Group p.l.c., or by analogous methods thereto.

As mentioned previously, the compound of formula (I) has been found to have blood-pressure lowering activity and bronchodilation activity. It is therefore useful in the treatment of hypertension and/or respiratory tract disorders. It is also believed to be of potential use in the treatment of other disorders hereinbefore referred to.

The present invention accordingly provides a pharmaceutical composition which comprises the compound of formula (I) and a pharmaceutically acceptable carrier. In particular, the present invention provides an anti-hypertensive or bronchodilatory pharmaceutical composition which comprises an effective amount of the compound of formula (I) and a pharmaceutically acceptable carrier.

The compositions are preferably adapted for oral administration. However, they may be adapted for other modes of administration, for example parenteral administration for patients suffering from heart failure. Other alternative modes of administration include sublingual or transdermal administration. A composition may be in the form of spray, aerosol or other conventional method of inhalation, for treating respiratory tract disorders.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

For transdemal administration, formulations suitable for topical administration may be employed, optionally containing penetration enhancers.

The compositions may contain from 0.1% to 99% by weight, preferably from 10-60% by weight, of the active material, depending on the method of administration.

The compound of formula (I) is believed to show a synergistic effect with ACE inhibitor or β-blocker antihypertensive agents and such combination products, for concomitant or sequential administration, are within the present invention.

The present invention further provides a method of prophylaxis or treatment of hypertension or respiratory tract disorders in mammals including man, which comprises administering to the suffering mammal an anti-hypertensive or bronchodilatory effective amount of the compound of formula (I).

An effective amount will depend on the severity of the disorder being treated and the weight of the sufferer. However, a unit dose form of a composition of the invention may contain from 0.05 to 50 mg of a compound of the invention and more usually from 0.1 to 5 mg, for example 0.5 to 2.5 mg such as 0.5, 1 or 2 mg. Such compositions may be administered from 1 to 6 times a day, more usually from 1 to 4 times a day, in a manner such that the daily dose is from 0.01 to 5 mg per kg body weight and more particularly from 0.1 to 3 mg/kg.

No toxicological effects are indicated at the aforementioned dosage ranges.

The present invention further provides the compound of formula (I) for use in the treatment or prophylaxis of hypertension and/or respiratory tract disorders.

The following example relates to the preparation of the compound of formula (I).

### Description 1

### a) (±)-trans-4-Amino-6-cyano-3,4-dihydro-2,2-dimethyl-2H-1-benzopyran-3-ol

Sodium hydride (80% dispersion in oil, 13.7g) was added in portions over 1h to a stirred solution of (±)-trans-3-bromo-6-cyano-3,4-dihydro-2,2-dimethyl-2H-benzopyran-4-ol (124.3g) in tetrahydrofuran (250ml) kept under a dry nitrogen atmosphere. The mixture was stirred for an additional 0.5h after which a solution of the 3,4-epoxide resulted. Ethanol (620ml) followed by 0.880 ammonium hydroxide (375ml) were added, and the resulting mixture stirred at 60-65°C for 12h before cooling to room temperature. The organic solvents were evaporated off and the aqueous residue acidified with 5N hydrochloric acid (125ml). The mixture was then washed well with dichloromethane (total used 1.0L) before basifying with 40% aq. sodium hydroxide (80ml). It was then re-extracted with dichloromethane (4 x 250ml) and the combined extracts washed once with brine and then dried (Na₂SO₄). Evaporation afforded the product as a gum which crystallised. This was broken up and triturated with a mixture of isopropyl ether and dichlormethane before filtering off and washing with further isopropyl ether. The product was dried under suction and finally under vacuum.
Yield: 83.5g (87%) m.p.116-117°C.
δ(CDCl₃): 1.21 (s, 3H); 1.51 (s, 3H); 2.10 (b, 3H); 3.30 (d, J = 10Hz, 1H); 3.65 (d, J = 10Hz, 1H), 6.82 (d, J = 8Hz, 1H); 7.42 (m, 1H) 7.74 (m, 1H)

### b) Resolution of (±)-trans-4-Amino-6-cyano-3,4-dihydro-2,2-dimethyl-2H-1-benzopyran-3-ol

The title compound (100g) was dissolved in isopropyl alcohol (500ml) with stirring and heating to 70°C. Water (250ml) was added followed by (+)-ammonium 3-bromo-camphor-9-sulphonate (150.5g). The mixture was stirred and warmed back to 70°C to effect dissolution. 5N Hydrochloric acid (80ml) was then added fairly rapidly until the mixture reached pH5. It was then cooled to 55°C before seeding with authentic crystalline product. The mixture was cooled to room temperature before filtering off the product and washing with a mixture of isopropyl alcohol (50ml) and water (25ml). After drying in air at 50°C the yield of 3-bromo-camphor-9-sulphonic acid salt of the (+)-isomer of the title compound was 75g (31%).
[α]_{D}²⁰ (c=1, MeOH) = + 88.9°, m.p. 288-291°C.
δ(d₆-DMSO): 0.81 (s, 3H); 1.07 (s, 3H); 1.15 (s, 3H); 1.10-1.25 (m, 1H); 1.45 (s, 3H); 1.66-1.88 (m, 2H); 2.05-2.20 (m, 1H), 2.36 (d, J = 14Hz, 1H); 2.83 (d, J = 14Hz, 1H); 2.97 (ss, J = 6,6Hz, 1H); 3.64 (dd, J = 6,10Hz, 1H); 4.30 (d, J = 10Hz, 1H); 5.00 (d, J = 6Hz, 1H); 6.42 (d, J = 6Hz, 1H); 7.04 (d, J = 8Hz, 1H); 7.76 (m, 1H); 8.07 (bs, 1H); 8.53 (bs, 3H).

The foregoing salt (75g) was dissolved in a solution of potassium hydroxide (10.3g) in water (50 ml) and the mixture extracted with dichloromethane (4 x 250ml). The combined extracts were washed once with brine and dried (Na₂SO₄). Evaporation afforded the (+)-(3S,4R) isomer of the title compound as a glassy solid (30.5g; 99%). Crystallisation from ethyl acetate-petrol afforded prisms of m.p. 85-86°C.
[α]_{D}²⁰ (c = 1, MeOH) + 82.4°.

### Description 2

### a) trans-4-Amino-2,2-dimethyl-6-ethylchroman-3-ol

2,2-Dimethyl-3,4-epoxy-6-ethylchroman (1.596 kg) was dissolved in 2-propanol (4 L) and ammonia (3.2 L) was added. The mixture was warmed gently under reflux using a condenser at -78°. This reflux was maintained for 5h and then the mixture was warmed slowly to 60° overnight. The solvent was then removed under reduced pressure and the residue was recrystallised from cyclohexane giving 1.19 kg (69%) of the required title amine.

### b) 4S-Ammonium-3,4-dihydro-2,2-dimethyl-6-ethyl-2H-1-benzopyran-3R-ol (+)-mandelate

(+)-Mandelic acid (0.57 kg) and trans-4-amino-2,2-dimethyl-6-ethylchroman-3-ol (0.75 kg) were dissolved in warm 2-propanol (18 L). The solution was concentrated to 12 L and crystallised giving the title product, 0.523 kg (41.3%).

### Example (Method 1)

### a) 6-Cyano-3,4-dihydro-2,2-dimethyl-4R-(2-oxopiperidin-1-yl)-2H-1-benzopyran-3S-ol

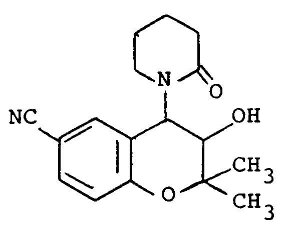

5-Chlorovaleryl chloride (8.88g) was added portionwise to a stirred solution of 4R-amino-6-cyano-3,4-dihydro-2,2-dimethyl-2H-1-benzopyran-3S-ol (8.33g) and triethylamine (5.8g) in 1-methyl-2-pyrrolidinone (30 ml) at ice-bath temperature under an atmosphere of dry nitrogen. The reaction mixture was allowed to warm to room temperature and stirred for a further 16h, then was cooled to -30°C, and potassium tert-butoxide (15.43g) added portionwise with vigorous stirring. The reaction mixture was allowed to warm to room temperature and was stirred for 30 min, then poured onto crushed ice, the pH was adjusted to 7 (dilute hydrochloric acid) and the resulting solid collected by filtration, washed with water and dried in vacuo to give the title product as a white solid (9.28g).

A further crop of product (0.75g) was obtained by extraction of the aqueous filtrate with ethyl acetate.
¹H-n.m.r. (CDCl₃) δ = 1.25 (s,3H); 1.52 (s,3H); 1.62-2.07 (m,4H); 2.47-2.66 (m,2H); 2.78-2.96 (m,1H); 3.05-3.24 (m,1H); 3.50 (br.s.,1H); 3.77 (d,J=11Hz,1H); 5.92 (d,J=11Hz,1H); 6.88 (d,J=9Hz,1H); 7.26 (d,J=2Hz,1H); 7.45 (d.d.,J=2,9Hz,1H).

### b) 3,4-Dihydro-2,2-dimethyl-6-formyl-4R-(2-oxopiperidin-1-yl)-2H-1-benzopyran-3S-ol

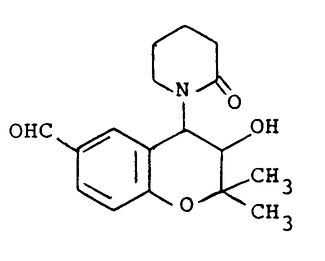

6-Cyano-3,4-dihydro-2,2-dimethyl-4R-(2-oxopiperidin-1-yl)-2H-1-benzopyran-3S-ol (9.23g), Raney Nickel (6.5g) and formic acid (75% v/v, 100 ml) were heated under reflux, with vigorous stirring, for 1h. The hot reaction mixture was filtered, the residue washed with hot ethanol and the combined filtrates were evaporated in vacuo. The residue was treated with dilute sodium hydrogen carbonate solution until alkaline (pH 9) and then extracted twice with ethyl acetate. The combined ethyl acetate extractions were washed with dilute hydrochloric acid, dilute sodium hydrogen carbonate, then brine, and dried (anhydrous sodium sulphate). Filtration and evaporation of the solvent gave a solid (8.94g). Recrystallization from ethyl acetate-methanol gave the title product as pale lemon-coloured crystals (6.96g); m.p. 226-229°C.

Column chromatography of the mother liquors (silica gel, gradient elution chloroform-methanol) gave a second crop of the title compound (1g).
¹H n.m.r. (CDCl₃) δ = 1.30 (s,3H); 1.56 (s,3H); 1.66-2.01 (m,4H); 2.49-2.74 (m,2H); 2.84-3.01 (m,1H); 3.03-3.21 (m,1H); 3.52 (brs,1H); 3.81 (d,J=11Hz,1H); 5.98 (d,J=11Hz,1H); 6.95 (d,J=9Hz,1H); 7.52 (d,J=2Hz,1H); 7.72 (dd,J=2,9Hz,1H); 9.86 (s,1H).

### c) 3,4-Dihydro-2,2-dimethyl-6-ethenyl-4R-(2-oxopiperidin-1-yl)-2H-1-benzopyran-3S-ol

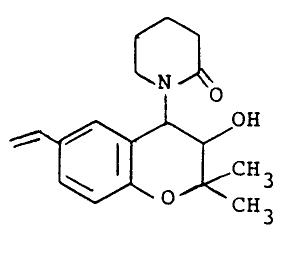

Potassium tert-butoxide (4.14g) was added portionwise to a stirred suspension of methyl triphenylphosphonium bromide (12.11g) in dry diethyl ether (100 ml). The mixture was ultrasonicated for 18h. 1-Methyl-2-pyrrolidinone (30 ml) was added and the mixture was ultrasonicated for a further 4h. 3,4-Dihydro-2,2-dimethyl-6-formyl-4R-(2-oxopiperidin-1-yl)-2H-1-benzopyran-3S-ol (7.84g) was then added and ultrasonication maintained for 1h. The solvent was evaporated in vacuo, and the residue partitioned between ethyl acetate and water. The aqueous phase was further extracted with ethyl acetate and the combined organic extracts washed with water, then brine and dried (anhydrous sodium sulphate). Filtration and evaporation of the solvent gave a gum. Column chromatography (silica gel - Kieselgel 60 - eluting with ethyl acetate) gave a white solid. Recrystallizations from ethyl acetate then isopropanol gave the title compound as a white crystalline solid (3g); m.p. 205-8°C.

A further crop of the title compound (2.2g) was obtained from the recrystallization mother liqueurs after removal of the residual starting material by treatment with excess hydroxylamine and separation by column chromatography.
¹H n.m.r. (CDCl₃) δ = 1.25 (s,3H); 1.50 (s,3H); 1.64-1.95 (m,4H); 2.48-2.73 (m,2H); 2.91-3.13 (m,2H); 3.19 (brs,1H); 3.79 (d,J=11Hz,1H); 5.13 (d,J=11Hz); 5.58 (d,J=18Hz,1H); 5.91 (d,J=9Hz,1H); 6.62 (dd,J=11,18Hz); 6.80 (d,J=9Hz,1H); 6.97 (d,J=2Hz,1H); 7.28 (dd,J=2Hz,1H).

### d) 3,4-Dihydro-2,2-dimethyl-6-ethyl-4R-(2-oxopiperidin-1-yl)-2H-1-benzopyran-3S-ol

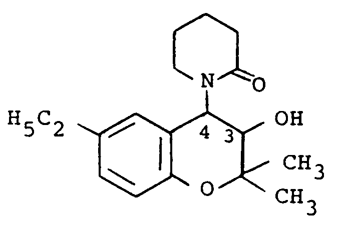

A mixture of 3,4-dihydro-2,2-dimethyl-6-ethenyl-4R-(2-oxopiperidin-1-yl)-2H-1-benzopyran-3S-ol (5.15g), 10% palladium on carbon (0.4g) in ethanol (200 ml) and ethyl acetate (50 ml) were stirred for 1h under a hydrogen atmosphere. The catalyst was removed by filtration and the solvent evaporated in vacuo. The residue was dissolved in ethyl acetate and dried (anhydrous sodium sulphate). The solution was filtered and then further filtered through a small pad of Kieselgel 60. Evaporation of the solvent gave a white solid which was recrystallised from diisopropyl ether to give the title compound as a white crystalline solid (4.5g); m.p. 140-1°C.
¹H n.m.r. (CDCl₃) δ = 1.18 (t,J=8Hz,3H); 1.25 (s,3H); 1.50 (s,3H); 1.62-1.96 (m,4H); 2.43-2.73 (m, containingquartet at 2.56, J=8Hz,4H); 2.89-3.14 (m,2H); 3.38 (brs,1H); 3.78 (d,J=11Hz,1H); 5.88 (d,J=11Hz,1H); 6.68-6.84 (m,2H); 7.00 (dd,J=2,9Hz,1H).

### Example (Method 2)

### 3,4-Dihydro-2,2-dimethyl-6-ethyl-4R-(2-oxopiperidin-1-yl)-2H-1-benzopyran-3S-ol

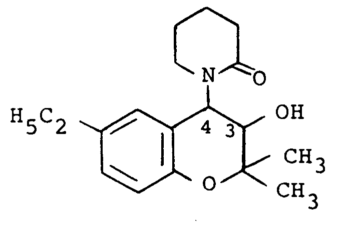

The (+) mandelate of Description 2b) (0.4 kg) was partitioned between toluene (1 L) and 2.5% sodium hydroxide solution (1.92 L). The aqueous layer was separated and washed with toluene (1 L). The combined organic layers were washed with water (2x0.5 L) and the solvent was removed under reduced pressure. The residue was dissolved in toluene (1 L) and triethylamine (0.164 L) and 5-chlorovaleroyl chloride (0.152 L) were added. A solution of potassium tert-butoxide (0.6 kg) in toluene (1.2 L) and 2-propanol (0.5 L) was added and the mixture was stirred overnight. Water (1.0 L) was added and the organic layer was separated. The aqueous layer was extracted with toluene (1.0 L) and the combined organic layers were washed with 1N hydrochloric acid (3x0.5 L) and water (4x0.5 L). The solvent was removed under reduced pressure and the solid obtained was triturated with hot di-isopropyl ether to give the title compound 0.275 kg (85%); m.p. 138-140°C. [α]_{D}²⁰ (c=1, MeOH) -97°.

### PHARMACOLOGICAL DATA

The compound of the invention was tested for activity in the following test methods.

### 1. Antihypertensive Activity

Systolic blood pressures were recorded by a modification of the tail cuff method described by I.M. Claxton, M.G. Palfreyman, R.H. Poyser, R.L. Whiting, European Journal of Pharmacology, 37, 179 (1976). A W+W BP recorder, model 8005 was used to display pulses. Prior to all measurements rats were placed in a heated environment (33.5 ± 0.5°C) before transfer to a restraining cage. The determination of blood pressure was the mean of 5 readings. Spontaneously hypertensive rats (ages 12-18 weeks) with systolic blood pressures >180 mmHg were considered hypertensive.

The compound of formula (I) of the Example showed a maximum fall in blood pressure of 19% at a dose of 0.05 mg/kg p.o.

### 2. Bronchodilator Activity

Male guinea pigs (300-600g) were stunned by a blow to the head and bled from the carotid artery. The trachea was exposed, dissected free of connective tissue, and transferred to oxygenated Krebs solution at 37°C. Next, spirals (2 per trachea) were prepared by cutting the whole trachea spirally along its longitudinal axis and then dividing this spiral lengthwise. Each preparation was mounted, using silk thread, in a 10ml organ bath filled with Krebs solution at 37°C and bubbled with 5% CO₂ with O₂. The resting tension of the preparations was set at 2g and changes in muscle tension were monitored isometrically by means of a UFI (2oz) force and displacement transducer (Ormed Ltd) connected to a Linseis pen recorder. All preparations were allowed to equilibrate for 60 minutes. During this equilibration period the preparations were washed by upward displacement at 15 minute intervals and, if necessary, the resting tension was readjusted to 2g using a mechanical micromanipulator system.

Once a steady resting tension had been obtained, the preparations were dosed simultaneously with the test compound (10⁻⁸-2x10⁻⁵M), and finally a maximum relaxation achieved by addition of 10⁻³M isoprenaline. The fall in tension evoked by the test compound was expressed as a percentage of the total relaxation evoked in the presence of 10⁻³ isoprenaline. Appropriate concentration-relaxation curves were then constructed and values for potency (IC₅₀) were obtained.

The compound of formula (I) of the example gave an IC₅₀ value of 0.23 µmol.

[The composition of Krebs solution is: sodium chloride 118.07mM, sodium hydrogen carbonate 26.19mM, potassium chloride 4.68mM, potassium orthophosphate 1.18mM, magnesium sulphate septahydrate 1.8mM and calcium chloride 2.52mM;pH ca. 7.45.]

## Claims (Claims for the following Contracting State(s): AT, BE, CH, DE, DK, FR, GB, GR, IT, LI, LU, NL, SE)

1. The (3S,4R)-isomer of a compound of formula (I): 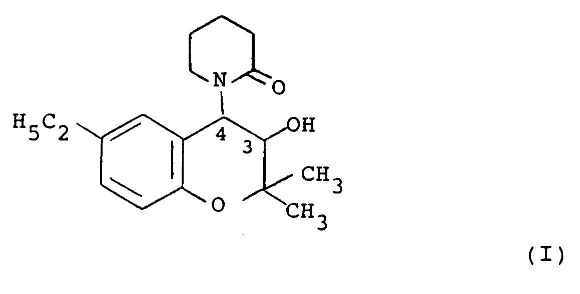 wherein the lactam and OH moieties are trans.

2. A process for the preparation of a compound according to claim 1, which process comprises the reaction of a compound of formula (II): 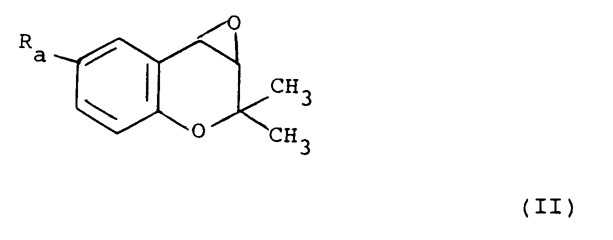 wherein Rₐ is ethyl or a group or atom convertible thereto; with either
i) a compound of formula (III): 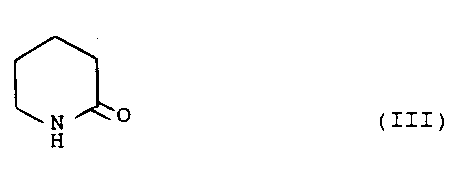 in the form of the piperidinone anion; or
ii) with ammonia to give a trans aminoalcohol of formula (IV): 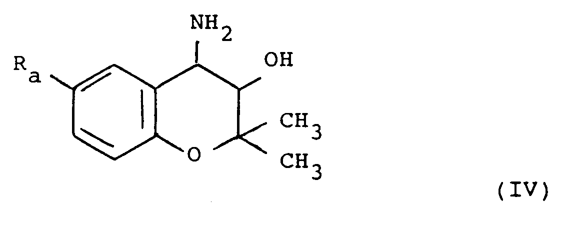 which is subsequently acylated with a compound of formula (V):
L₁(CH₂)₄COL₂ (V)
wherein L₁ and L₂ are leaving groups; and thereafter cyclising the resulting compound where necessary;
and thereafter converting Rₐ (when other than ethyl) to ethyl.

3. 3,4-Dihydro-2,2-dimethyl-6-ethenyl-4R-(2-oxopiperidin-1-yl)-2H-1-benzopyran-3S-ol.

4. A pharmaceutical composition comprising a compound according to claim 1 and a pharmaceutically acceptable carrier.

5. A compound according to claim 1 for use as an active therapeutic substance.

6. A compound according to claim 1 for use as a potassium channel activator in the treatment of disorders asociated with smooth muscle contraction.

7. A compound according to claim 1 for use in the treatment of hypertension and/or respiratory tract disorders.

8. Use of a compound according to claim 1 in the manufacture of a medicament for use as a potassium channel activator in the treatment of disorders associated with smooth muscle contraction.

9. Use of a compound according to claim 1 in the manufacture of a medicament for use in the treatment of hypertension and/or respiratory tract disorders.

## Claims (Claims for the following Contracting State(s): ES)

1. A process for the preparation of the (3S,4R)-isomer of a compound of formula (I): 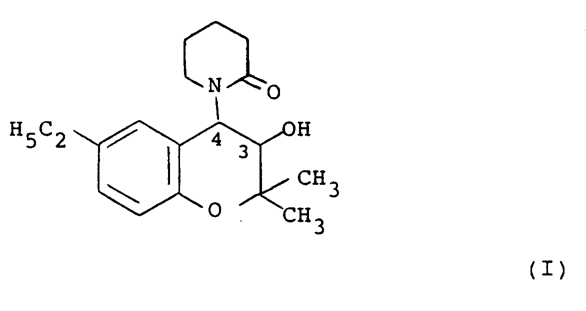 wherein the lactam and OH moieties are trans; which process comprises the reaction of a compound of formula (II): 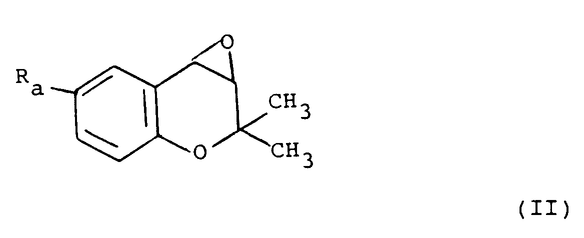 wherein Rₐ is ethyl or a group or atom convertible thereto; with either
i) a compound of formula (III): 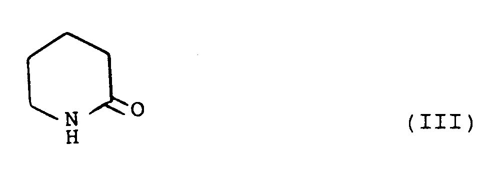 in the form of the piperidinone anion; or
ii) with ammonia to give a trans aminoalcohol of formula (IV): 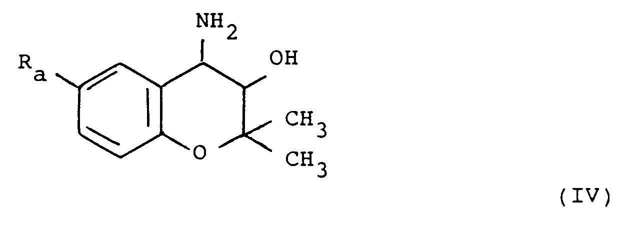 which is subsequently acylated with a compound of formula (V):
L₁(CH₂)₄COL₂ (V)
wherein L₁ and L₂ are leaving groups; and thereafter cyclising the resulting compound where necessary;
and thereafter converting Rₐ (when other than ethyl) to ethyl.

2. 3,4-Dihydro-2,2-dimethyl-6-ethenyl-4R-(2-oxopiperidin-1-yl)-2H-1-benzopyran-3S-ol.

3. Use of a compound according to claim 1 in the manufacture of a medicament for use as a potassium channel activator in the treatment of disorders associated with smooth muscle contraction.

4. Use of a compound according to claim 1 in the manufacture of a medicament for use in the treatment of hypertension and/or respiratory tract disorders.

## Patentansprüche (Patentansprüche für folgende(n) Vertragsstaat(en): AT, BE, CH, DE, DK, FR, GB, GR, IT, LI, LU, NL, SE)

1. (3S,4R)-Isomer der Verbindung der Formel (I): 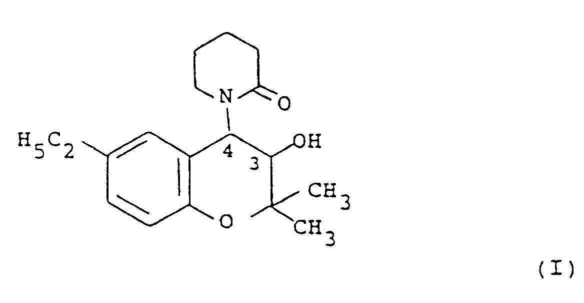 wobei die Lactam- und OH-Einheiten in trans-Stellung sind.

2. Verfahren zur Herstellung der Verbindung nach Anspruch 1, wobei dieses Verfahren umfaßt: Umsetzung einer Verbindung der Formel (II): 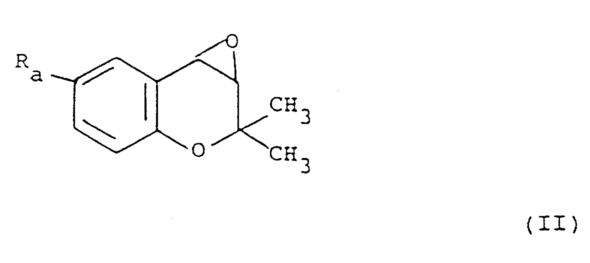 in der Rₐ eine Ethylgruppe oder eine Gruppe oder ein Atom ist, die in eine Ethylgruppe überführt werden können; mit entweder
i) einer Verbindung der Formel (III): 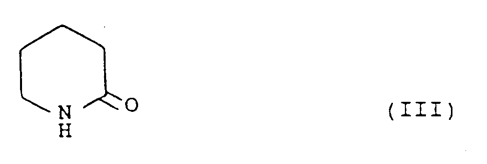 in Form des Piperidinon-Anions; oder
ii) mit Ammoniak, wodurch der trans-Aminoalkohol der Formel (IV) erhalten wird: 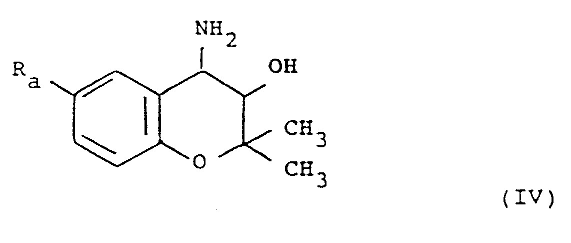 der anschließend mit einer Verbindung der Formel (V) acyliert wird:
L₁(CH₂)₄COL₂ (V)
in der L₁ und L₂ Austrittsgruppen sind; und falls erforderlich anschließende Cyclisierung der entstandenen Verbindung;
und anschließende Umwandlung von Rₐ (falls von einer Ethylgruppe verschieden) in eine Ethylgruppe.

3. 3,4-Dihydro-2,2-dimethyl-6-ethenyl-4R-(2-oxopiperidin-1-yl)-2H-1-benzopyran-3S-ol.

4. Arzneimittel, umfassend eine Verbindung nach Anspruch 1 und einen pharmazeutisch verträglichen Träger.

5. Verbindung nach Anspruch 1 zur Verwendung als therapeutischer Wirkstoff.

6. Verbindung nach Anspruch 1 zur Verwendung als Kaliumkanalaktivator bei der Behandlung von Störungen in Verbindung mit der Kontraktion der glatten Muskulatur.

7. Verbindung nach Anspruch 1 zur Verwendung bei der Behandlung von Hypertonie und/oder Störungen der Atemwege.

8. Verwendung der Verbindung nach Anspruch 1 zur Herstellung eines Medikamentes zur Verwendung als Kaliumkanalaktivator bei der Behandlung von Störungen in Verbindung mit der Kontraktion der glatten Muskulatur.

9. Verwendung der Verbindung nach Anspruch 1 zur Herstellung eines Medikamentes zur Verwendung bei der Behandlung von Hypertonie und/oder Störungen der Atemwege.

## Patentansprüche (Patentansprüche für folgende(n) Vertragsstaat(en): ES)

1. Verfahren zur Herstellung des (3S,4R)-Isomers einer Verbindung der Formel (I): 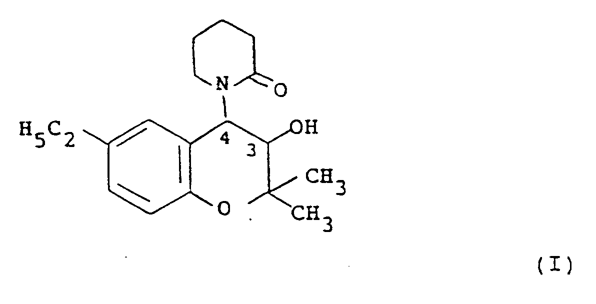 worin die Lactam- und OH-Einheiten in trans-Stellung sind; wobei dieses Verfahren umfaßt:
Umsetzung der Verbindung der Formel (II): 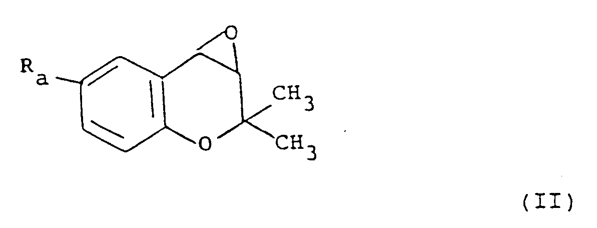 in der Rₐ eine Ethylgruppe oder eine Gruppe oder ein Atom ist, die in eine Ethylgruppe überführt werden können; mit entweder
i) einer Verbindung der Formel (III): 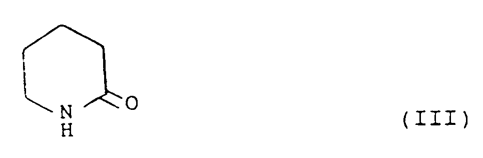 in Form des Piperidinon-Anions; oder
ii) mit Ammoniak, wodurch der trans-Aminoalkohol der Formel (IV) erhalten wird: 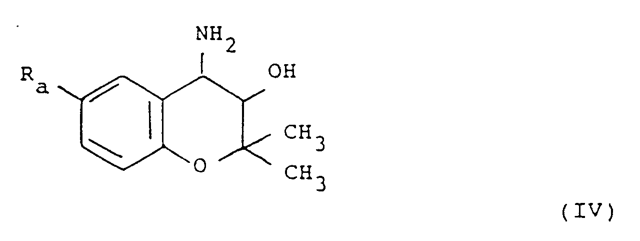 die anschließend mit einer Verbindung der Formel (V) acyliert wird:
L₁(CH₂)₄COL₂ (V),
in der L₁ und L₂ Austrittsgruppen darstellen; und falls erforderlich anschließende Cyclisierung der entstandenen Verbindung;
und anschließende Umwandlung von Rₐ (falls von einer Ethylgruppe verschieden) in eine Ethylgruppe.

2. 3,4-Dihydro-2,2-dimethyl-6-ethenyl-4R-(2-oxopiperidin-1-yl)-2H-1-benzopyran-3S-ol.

3. Verwendung einer Verbindung nach Anspruch 1 zur Herstellung eines Medikamentes zur Verwendung als Kaliumkanalaktivator bei der Behandlung von Störungen in Verbindung mit der Kontraktion der glatten Muskulatur.

4. Verwendung einer Verbindung nach Anspruch 1 bei der Herstellung eines Medikamentes zur Verwendung bei der Behandlung von Hypertonie und/oder Störungen der Atemwege.

## Revendications (Revendications pour l'(les) Etat(s) contractant(s) suivant(s): AT, BE, CH, DE, DK, FR, GB, GR, IT, LI, LU, NL, SE)

1. Isomère (3S, 4R) d'un composé de formule (I) : 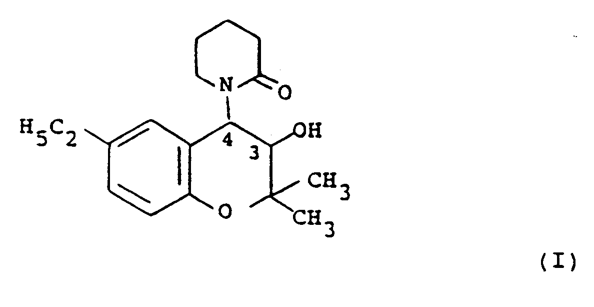 dans lequel les entités lactame et OH sont trans.

2. Procédé de préparation d'un composé suivant la revendication 1, lequel procédé comprend la réaction d'un composé de formule (II) : 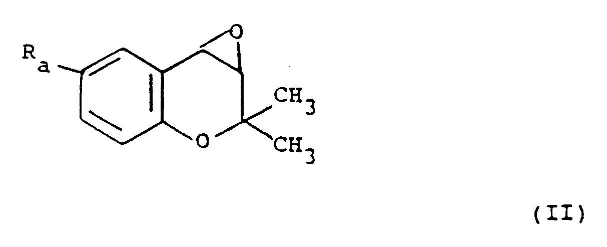 dans lequel Rₐ est un groupe éthyle ou un groupe ou atome convertible en éthyle, avec,
(i) soit un composé de formule (III) 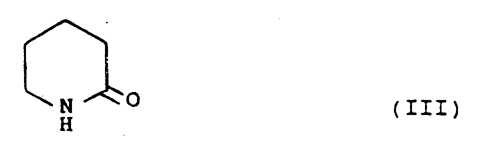 sous la forme de l'anion pipéridinone; soit
(ii) avec l'ammoniaque pour donner un trans amino-alcool de formule (IV): 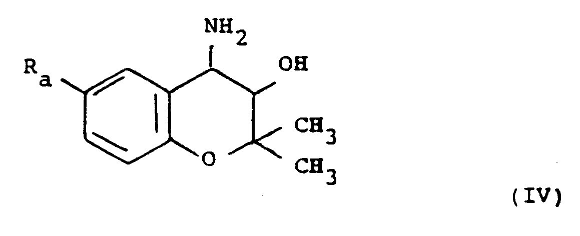 qui est ensuite acylé avec un composé de formule (V) :
L₁(CH₂)₄COL₂ (V)
dans lequel L₁ et L₂ sont des groupes partants; et ensuite, la cyclisation du composé obtenu, si nécessaire;
et ensuite, la conversion de Rₐ (si autre qu'un groupe éthyle), en un groupe éthyle.

3. 3,4-Dihydro-2,2-diméthyl-6-éthènyl-4R-(2-oxopipéridin-1-yl)-2H-1-benzopyran-3S-ol.

4. Composition pharmaceutique comprenant un composé suivant la revendication 1, et un véhicule, acceptable du point de vue pharmaceutique.

5. Composé suivant la revendication 1, pour l'utilisation en tant que substance thérapeutique active.

6. Composé suivant la revendication 1, pour l'utilisation en tant qu'activateur du transport du potassium, dans le traitement des troubles associés à la contraction des muscles lisses.

7. Composé suivant la revendication 1, pour l'utilisation dans le traitement de l'hypertension et/ou des troubles du tractus respiratoire.

8. Utilisation d'un composé suivant la revendication 1, dans la fabrication d'un médicament pour l'emploi comme activateur du transport du potassium dans le traitement des troubles associés à la contraction des muscles lisses.

9. Utilisation d'un composé suivant la revendication 1, dans la fabrication d'un médicament pour l'emploi dans le traitement de l'hypertension et/ou des troubles du tractus respiratoire.

## Revendications (Revendications pour l'(les) Etat(s) contractant(s) suivant(s): ES)

1. Procédé de préparation de l'Isomère (3S, 4R) d'un composé de formule (I) : 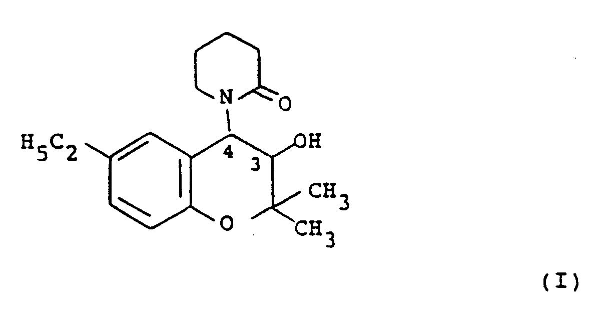 dans lequel les entités lactame et OH sont trans; lequel procédé comprend la réaction d'un composé de formule (II) : 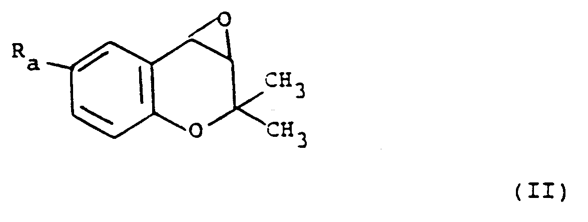 dans lequel Rₐ est un groupe éthyle ou un groupe ou atome convertible en éthyle, avec,
(i) soit un composé de formule (III) 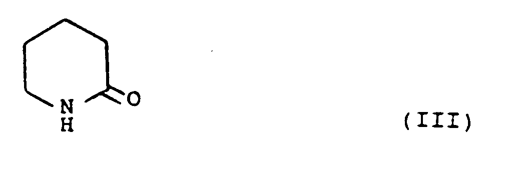 sous la forme de l'anion pipéridinone; soit
(ii) avec l'ammoniaque pour donner un trans amino-alcool de formule (IV): 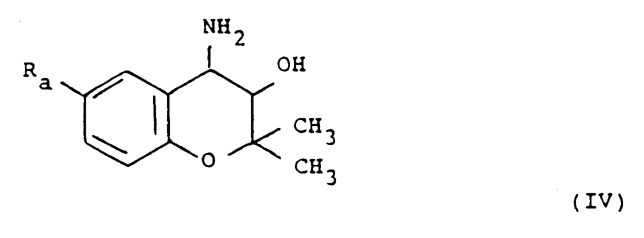 qui est ensuite acylé avec un composé de formule (V) :
L₁(CH₂)₄COL₂ (V)
dans lequel L₁ et L₂ sont des groupes partants; et ensuite, la cyclisation du composé obtenu, si nécessaire;
et ensuite, la conversion de Rₐ (si autre qu'un groupe éthyle), en un groupe éthyle.

2. 3,4-Dihydro-2,2-diméthyl-6-éthènyl-4R-(2-oxopipéridin-1-yl)-2H-1-benzopyran-3S-ol.

3. Utilisation d'un composé suivant la revendication 1, dans la fabrication d'un médicament pour l'emploi comme activateur du transport du potassium dans le traitement des troubles associés à la contraction des muscles lisses.

4. Utilisation d'un composé suivant la revendication 1, dans la fabrication d'un médicament pour l'emploi dans le traitement de l'hypertension et/ou des troubles du tractus respiratoire.
